# EUROPÄISCHE PATENTSCHRIFT

(11) **EP 0 628 554 B1**
(45) Veröffentlichungstag und Bekanntmachung des Hinweises auf die Patenterteilung: **02.08.2000**
(21) Anmeldenummer: 94108038.4
(22) Anmeldetag: 25.05.1994
(51) Int. Cl.: C07D 317/36, C07D 317/38

(54) **Verfahren zur Herstellung von Propylenglykolcarbonat**
Process for the preparation of propylene glycol carbonate
Procédé de préparation de carbonate de propylène glycol

(30) Priorität: 07.06.1993 DE 4318892
(43) Veröffentlichungstag der Anmeldung: 14.12.1994
(73) Patentinhaber: BAYER AG, 51368 Leverkusen (DE)
(72) Erfinder: Wagner, Paul, Dr., D-40597 Düsseldorf (DE); Mendoza-Frohn, Christine, Dr., D-40699 Erkrath (DE); Buysch, Hans-Josef, Dr., D-47809 Krefeld (DE)

(56) Entgegenhaltungen:
- EP-A- 0 546 428
- SU-A- 172 342
- PATENT ABSTRACTS OF JAPAN vol. 6, no. 197 (C-128)(1075) 6. Oktober 1982 & JP-A-57 106 631 (NIPPON SHOKUBAI) 2. Juli 1982

## Beschreibung

Die vorliegende Erfindung betrifft ein Verfahren zur kontinuierlichen katalytischen Herstellung von Propylenglykolcarbonat (PGC) aus Propylenoxid (POX) und Kohlendioxid (CO₂), das gekennzeichnet ist durch eine adiabate und besonders schonende, energie- und materialsparende Verfahrensweise.

Für die Herstellung von PGC sind viele Vorschläge gemacht worden, Diese beziehen sich in erster Linie auf die Verwendung bestimmter Katalysatoren. Ein Verfahren zur Herstellung von PGC bzw. Ethylenglykolcarbonat (EGC) aus POX bzw. Ethylenoxid (EOX) und CO₂ wird in Chem. Ing. Techn. 43 (1971), 903 beschrieben. Dieses Verfahren liegt auch der Veröffentlichung in Fette, Seifen, Anstrichmittel 73, (1971), 396 zugrunde, wo jedoch speziell nur auf die Herstellung von EGC eingegangen wird, Für die Herstellung von EGC wird dort eine adiabate Temperaturführung als technisch nicht realisierbar angesehen (loc. cit. 398). Im dort beschriebenen Verfahren werden CO₂ und EOX bei 80 bar und 190 bis oberhalb von 200°C in einem mit Ethylenglykolcarbonat gefüllten Reaktor miteinander umgesetzt und die Reaktionswärme mit Hilfe eines im Gegenstrom zirkulierenden Wärmeträgers abgeführt, der wiederum mit Wasser gekühlt wird. Bei dieser Art der Reaktionsführung erhält man Spitzentemperaturen bis zu 220°C im Reaktor, die produktschädigend wirken, worauf in der angegebenen Publikation ausdrücklich verwiesen wird (S. 397), und die insbesondere bei technischen Großanlagen schwer zu beherrschen sein dürften. Die gesamte Reaktionsenergie wird hierbei ungenutzt abgeführt. Da die Umsetzung von POX mit CO₂ zu PGC eine ähnlich hohe Wärmetönung aufweist wie die von EOX mit CO₂ zu EGC und sich Temperaturspitzen ebenfalls produktschädigend auf das PGC auswirken, lassen sich die Aussagen aus Fette, Seifen (loc. cit.) auch auf die Herstellung von PGC aus POX und CO₂ übertragen. Eine adiabate Temperaturführung ist im Gegensatz zu der in Fette, Seifen (loc. cit.) und Chem. Ing. Techn. (loc. cit.) beschriebenen Verfahrensweise dadurch gekennzeichnet, daß die gesamte freiwerdende Reaktionswärme durch das Reaktionsgemisch selbst aufgenommen wird; dies führt bei exothermen Reaktionen zu einer Erhöhung der Temperatur des Reaktionsgemisches.

In dem Verfahren, das in US 4 314 945 beschrieben ist, werden als Reaktoren für die Herstellung von Alkylencarbonaten aus Alkylenoxiden und CO₂ unterschiedliche Kombinationen von Strömungsrohren und Umpumpreaktoren zugrundegelegt, die bei 10-50 bar und 100 bis max. 200°C betrieben werden. Das entsprechende Carbonat dient in allen Reaktorteilen als Reaktionsmedium und stellt dort jeweils 85-99,6 Gew.-% des Reaktorinhalts,

Das Alkylenoxid wird in die erste Reaktionszone einer solchen Kombination gemeinsam mit dem in dem Carbonat gelösten Katalysator dosiert.

Die Zuspeisung des CO₂ erfolgt bei der in US 4 314 945 bevorzugten Kombination eines Umpumpreaktors mit nachgeschalteten Strömungsrohren an einer anderen, entfernten Stelle des Reaktors. Gerade im ersten Reaktor einer solchen Kombination, wo die lokale Alkylenoxid-Konzentration in der unmittelbaren Umgebung der Dosierstelle noch besonders hoch ist, wirkt sich die gewählte Dosierform nachteilig auf die Selektivität aus: das Alkylenoxid kann, begünstigt durch die hohe lokale Katalysatorkonzentration und die niedrigere CO₂-Konzentration, zu Acetaldehyd umlagern und dient so als Grundlage für Polykondensationsreaktionen.

Nachteilig kommt hinzu, daß CO₂ lediglich im leichten Überschuß gegenüber dem Katalysator (und nicht gegenüber dem Alkylenoxid) zugespeist wird.

Als Bauteile für die Kombinationen, die einen Reaktor darstellen, werden in US 4 314 945 einerseits Umpumpreaktoren - bestehend aus einem durch einen Rührer oder durch das einströmende Gas bzw. Flüssigkeit durchmischten Rührkessel mit äußerem Umlauf und Wärmeaustauscher zur Produktkühlung - und andererseits mit adiabater Temperaturführung und einem anschließenden Kühler betriebene oder selbst mit einem Kühler versehene Strömungsrohre verwendet.

Ein Umpumpreaktor ist dadurch gekennzeichnet, daß bei exothermen Reaktionen mit hohen Umpumpraten gearbeitet wird, wobei während der Reaktion im Reaktor und im zugehörigen Wärmeaustauscher die Reaktionswärme abgeführt wird. Diese Verfahrensweise ist völlig verschieden von der adiabaten Fahrweise, bei der die Reaktion bei einem Durchgang durch den Reaktor beendet ist und die gesamte Reaktionswärme vom Reaktionsmedium aufgenommen wird.

Den Reaktorbauteilen in US 4.314.945 ist, unabhängig von ihrer Anzahl und Reihenfolge innerhalb der Kombination, gemeinsam, daß in ihnen jeweils nur ein Teilumsatz des Alkylenoxids stattfinden kann, da sonst das Wärmeproblem der stark exothermen Umsetzung des Alkylenoxids mit dem CO₂ nicht beherrscht wird.

Nur durch eine aufwendige Verschaltung dieser jeweils mit Kühlem versehenen Einzelbauteile mit Teilumsatz kann insgesamt ein Umsatz an Alkylenoxid von mindestens etwa 99,5 % und eine Carbonatselektivität von mindestens etwa 99 % erhalten werden.

Eine Nutzung der fühlbaren Wärme der Reaktion im Prozeß selbst oder zur Gewinnung von Dampf wird in US 4.314.945 nicht beschrieben.

Auch aus SU-A-172 342 ist bereits ein Verfahren zur Herstellung von Alkylenglykolcarbonaten durch kontinuierliche Umsetzung der entsprechenden Alkylenoxide mit CO₂ bekannt. Das Verfahren läuft in Gegenwart eines geringen Überschusses an Alkylenglykolcarbonat als Reaktionsmedium bei einem Druck von 100 bar unter adiabatischer Temperaturführung ab. Das am Kopf des Reaktors entnommene Produkt wird unmittelbar kondensiert und rektifiziert. Nachteilig ist, daß es im Reaktor durch die bei der Reaktion freigesetzte Energie zu einem explosionsartigen Anstieg der Temperatur von 180 auf 290°C, d.h. um 110°C kommt.

Es bestand der Wunsch, ein Verfahren zu entwickeln, daß einerseits schädliche Temperaturspitzen vermeidet und andererseits die Reaktionsenergie soweit wie möglich nutzt; eine solche Nutzung kann im Rahmen des erfindungsgemäßen Verfahrens selbst, beispielsweise zur Aufarbeitung des Rohprodukts oder zur Erzeugung von Heizdampf für andere Verfahren, erfolgen.

Es wurde ein Verfahren zur katalytischen Herstellung von Propylenglykolcarbonat (PGC) durch Umsetzung von Propylenoxid (POX) und Kohlendioxid in PGC als Reaktionsmedium bei erhöhter Temperatur und erhöhtem Druck und Abtrennung des gebildeten PGC vom Katalysator gefunden, das dadurch gekennzeichnet ist, daß
a) das Verfahren kontinuierlich und adiabat bei einem Druck von 2 bis 200 bar, bevorzugt 5 bis 80 bar, besonders bevorzugt 8 bis 60 bar und innerhalb eines Temperaturbereiches von 110 bis 200°C, bevorzugt 110 bis 190°C, besonders bevorzugt 110 bis 180°C bei einer adiabaten Temperatursteigerung von 2 bis 80°C durchgeführt wird, wobei die Eingangstemperatur so gewählt wird, daß die adiabate Temperatursteigerung innerhalb des genannten Temperaturbereiches bleibt,
b) das als Reaktionsmedium dem Reaktor pro Zeiteinheit zulaufende PGC das 7 bis 350-fache des in dieser Zeiteinheit gebildeten PGC beträgt,
c) 1,01 bis 1,5 Mol, bevorzugt 1,01 bis 1,4 Mol, besonders bevorzugt 1,01 bis 1,35 Mol Kohlendioxid pro Mol POX eingesetzt werden und an allen Stellen des Reaktors ein Kohlendioxid-Überschuß aufrechterhalten wird und
d) 80 bis 98 Gew.-%, bevorzugt 85 bis 97 Gew,-%, des gesamten Reaktionsgemisches an den Eingang des Reaktors zurückgeführt werden und der Rest auf PGC aufgearbeitet wird.

Die Ausgangsprodukte POX und CO₂ werden in der Regel in einer Reinheit von mindestens 99 % eingesetzt. Es ist jedoch gleichfalls möglich, POX und CO₂ in geringerer Reinheit einzusetzen, wenn der Rest zu 100 % aus inerten Stoffen, wie beispielsweise Kohlenwasserstoffen, Kohlenmonoxid oder Stickstoff, besteht. Dies gilt besonders für CO₂, das aus verschiedenen Quellen stammen kann, beispielsweise aus natürlichen Quellen oder aus Anlagen zur Erzeugung von Wassergas, Kohlenmonoxid oder Reformern und dementsprechend von geringerer Reinheit ist. Solche Inertgase sind jedoch zweckmäßigerweise in einem Anteil von nicht mehr als 10 Vol.-%, bevorzugt nicht mehr als 5 Vol.-%, besonders bevorzugt nicht mehr als 3 Vol.-% anwesend.

Als Katalysatoren können praktisch alle bisher vorgeschlagenen verwendet werden, wie Alkali- und Erdalkalibromide und -iodide, Guanidine und deren Hydrobromide oder Hydroiodide, Tetraalkylammoniumbromide und -iodide, Phosphoniumbromide und -iodide, Pyridiniumhalogenide, Sulfonium-, Stibonium- und Arsoniumhalogenide, Zink- und Bleihalogenide, Alkylzinnverbindungen oder Gemische aus Alkalihalogeniden mit Halogeniden zweiwertiger Metallionen (FR 1 538 576; BE 872 960; EP 297 647; US 3 535 342; US 2 773 070; US 2 994 705; DE 15 43 555; US 3 535 41 ; BE 798 171; BE 872 959; DE-OS 32 44 456; GB 2 098 985; EP 133 763; EP 180 387; J. Org. Chem. 45 (1980), 3735; Chem. Lett. 1979, 573; DE-OS 41 05 554). In bevorzugter Weise werden als Katalysatoren eingesetzt: Alkalibromide und -iodide, Tetraalkylammoniumbromide und -iodide, Phosphoniumhalogenide, Guanidiniumhalogenide und Gemische aus Alkalihalogeniden mit Halogeniden zweiwertiger Metalle.

Das Verfahren wird im Temperaturbereich von 110 bis 200°C, bevorzugt 110 bis 190°C, besonders bevorzugt 110 bis 180°C durchgeführt. Der Druck für das erfindungsgemäße Verfahren beträgt 2 bis 200 bar, bevorzugt 5 bis 80 bar, besonders bevorzugt 8 bis 60 bar; bei Temperaturen im oberen Teil des angegebenen Bereiches werden auch Drucke im oberen Teil des angegebenen Bereiches benutzt und umgekehrt.

Das erfindungsgemäße Verfahren wird mit adiabater Temperaturführung durchgeführt, so daß die adiabate Temperatursteigerung 2 bis 80°C beträgt. Die Eingangstemperatur wird hierbei so gewählt, daß auch bei voller Ausnutzung der gewählten adiabaten Temperatursteigerung die Obergrenze des angegebenen Temperaturbereiches für das gesamte Verfahren nicht überschritten wird.

Erfindungsgemäß wird an allen Stellen des Reaktors ein CO₂-Überschuß über den anderen Reaktionspartner POX aufrechterhalten. Hierzu werden 1,01 bis 1,5 Mol, bevorzugt 1,01 bis 1,4 Mol, besonders bevorzugt 1,01 bis 1,35 Mol CO₂ pro Mol POX eingesetzt.

PGC als Reaktionsmedium liegt stets in großem Überschuß über das neu gebildete PGC vor. So läuft dem Reaktor pro Zeiteinheit das 7- bis 350-fache der in dieser Zeiteinheit gebildete PGC als Umlauf-PGC, das auch den Katalysator enthält, zum Dieser große Überschuß wird weiterhin dadurch aufrechterhalten, daß 80 bis 98 Gew.-%, bevorzugt 85 bis 97 Gew.-%, des gesamten Reaktionsgemisches an den Eingang des Reaktors zurückgeführt werden und lediglich der Rest zu 100 % abgezogen und auf reines PGC aufgearbeitet wird.

Es ist ein weiteres Kennzeichen des erfindungsgemäßen Verfahrens, daß die durch die adiabate Temperaturführung entstandene fühlbare Wärme des Reaktionsgemisches genutzt wird. Die wichtigste dieser Nutzungsmöglichkeiten ist hierbei die zur Aufarbeitung des Reaktionsgemisches auf reines PGC. In der Regel ist die erreichte fühlbare Wärme des Reaktionsproduktes jedoch so groß, daß darüber hinaus noch Heizdampf erzeugt werden kann, der anderen (endothermen) Verfahren zugeführt werden kann. In bevorzugter Weise wird hierbei zur Gewinnung der fühlbaren Wärme eine Abkühlung des Reaktionsgemisches um 2 bis 80°C vorgenommen.

Zur Vermeidung unerwünschter Temperaturspitzen wird im Reaktor eine intensive Durchmischung aufrechterhalten.

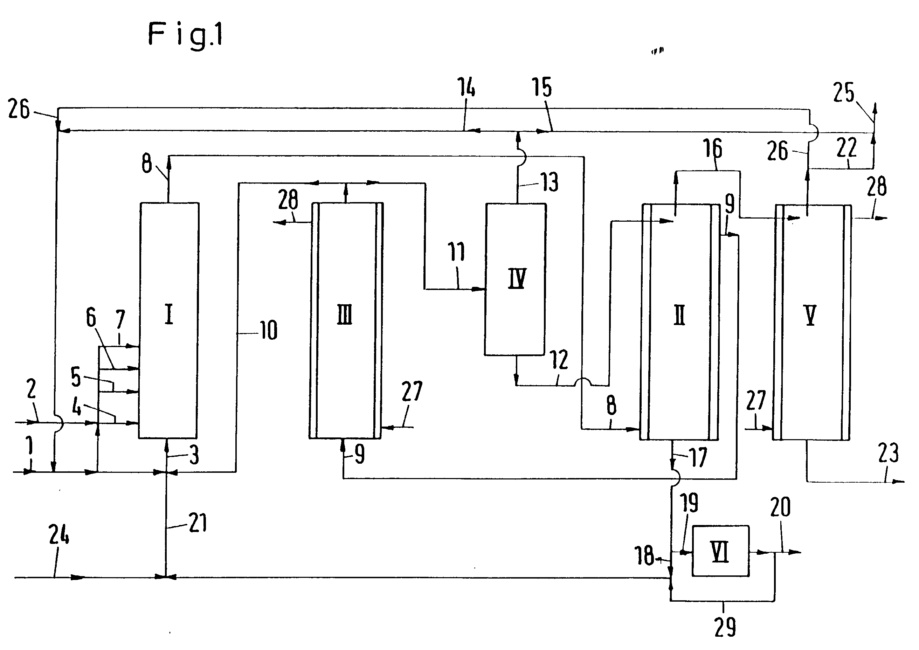

Weitere Einzelheiten des erfindungsgemäßen Verfahren werden im folgenden beschrieben, wozu außerdem auf die beigefügte Fig. 1 verwiesen wird, die beispielhaft eine mögliche Ausführungsform des erfindungsgemäßen Verfahrens zeigt. Die Aufarbeitung des Reaktionsgemisches wird in diesem Beispiel destillativ durchgeführt. Selbstverständlich sind auch andere Ausführungsvarianten als die der Fig. 1 möglich.

Der Reaktor (I) ist ein gut isolierter Apparat, beispielsweise ein gut isolierter Rohrreaktor; er enthält Einbauten die eine fortlaufende gute Verteilung der eindosierten Gase POX und CO₂ gewährleisten und an allen Stellen intensive Durchmischung erzeugen. Solche Einbauten, die dem Fachmann bekannt sind, sind beispielsweise Lochplatten, Loch- und Prallplattenböden, Rohrverteiler (Einsteck- und Ringverteiler), Zweistoffdüsen, Strahldüsen, Düsenböden, Düsen, besonders solche gemäß DE-OS 37 36 988 und DE-OS 37 44 001, technisch übliche Begasungsböden, Sintermetallfritten, geschlossene Gasverteilerböden mit Durchtrittsöffnungen für das PGC, rotierende Begasungsapparate, Impingment Aerator-Elemente (Perrys Chemical Engineers' Handbook 1984 S. 18.57-18.70), Mischerelemente, Blecheinbauten zur Erhöhung der Turbulenz, Segment- und Kreisringumlenkbleche. Diese Einbauten können auch in Kombination miteinander eingesetzt werden. Allgemein sollten solche Einbauten neben einer guten Durchmischung der Strömung (Makrovermischung) und der feinen Gasverteilung einen möglichst großen Anteil hochfrequenter Turbulenzelemente im Dissipationsbereich erzeugen. Bevorzugte Einbauten sind Lochböden, Rohrverteiler, Zweistoffdüsen, Strahldüsen, Düsen, besonders solche nach DE-OS 37 36 988 und DE-OS 37 44 001, geschlossene Gasverteiler, Impingment Aerator-Elemente und Mischerelemente.

CO₂ wird im erfindungsgemäßen Verfahren gemäß Ausführungsvariante von Fig. 1 bei (1) und POX bei (2) zugeführt.

Da es für die hohe Selektivität der erfindungsgemäßen Umsetzung von Bedeutung ist, daß POX überall im Reaktor auf CO₂ trifft und selber nirgendwo im Überschuß oder auch nur in hohen Konzentrationen auftritt, wird CO₂ in Strömungsrichtung vor dem POX in den Reaktor eingespeist. Diese vor dem POX vorgenommene Einspeisung von CO₂ wird zumindest teilweise vorgenommen, während der Rest an CO₂ in einer oder mehreren Teilmengen an weiteren Stellen des Reaktors so eingespeist wird, daß CO₂ stets im Überschuß über das POX vorhanden ist. In einer bevorzugten Variante wird gemäß Fig. 1 dem in den Reaktor strömenden PGC (3), das bereits den Katalysator enthält, schon vor dem Eintritt in den Reaktor CO₂ vollständig oder wenigstens teilweise zugemischt. In bevorzugter Weise betrifft diese Vorabzumischung von CO₂ die Überschußmenge von CO₂ im Umfang des oben beschrieben über die Menge des POX hinausgehenden CO₂-Überschusses von 1 bis 50 Mol-%. Erst an einer späteren Eintrittstelle (4) wird POX zugesetzt, bevorzugt im Gemisch mit CO₂; ein solches Gemisch besteht in besonders bevorzugter Weise aus äquimolaren Mengen POX und CO₂. In weiterhin bevorzugter Weise wird ein POX/CO₂-Gemisch an mindestens zwei Einspeisungsstellen in den Reaktor eingespeist. Eine solche mindestens zweite Einspeisungsstelle ist (5), weitere Einspeisungsstellen können (6) und (7) sowie noch weitere sein. Diese eingespeisten Gase werden sodann mit Hilfe der obengenannten Einbauten (Verteilungsorgane) gleichmäßig im Reaktionsmedium verteilt, Die größere Anzahl der Einspeisungsstellen (4), (5), (6), (7) und weitere vermeidet örtliche Überkonzentrationen und örtliche Temperaturspitzen im Reaktor.

Nach dem Abklingen der Hauptreaktion im Reaktor (I) strömt das Reaktionsgemisch (8) zur Nutzung der fühlbaren Wärme in den Heizmantel des Schnellverdampfers (II) und von dort als abgekühltes Reaktionsgemisch (9) zur weiteren Nutzung durch einen Wärmeaustauscher (III) in welchem aus Wasser (27) Heizdampf (28) gewonnen wird. Der Ablauf (8) des Reaktors kann jedoch auch zunächst durch den Wärmetauscher (III) und dann durch den Heizmantel des Schnellverdampfers (II) geführt werden. An die Stelle des Heizdampf erzeugenden Wärmetauschers (III) kann auch ein zu beheizender Reaktor eines völlig anderen Verfahrens treten, dessen Reaktion endotherm verläuft. Man kann jedoch den Ablauf (8) auch vor Eintritt in den Schnellverdampfer (II) in Teilströme, entsprechend (10) und (11), aufteilen.

Für den Reaktorablauf (8) und das bereits etwas abgekühlte Reaktionsgemisch (9) bedeuten die Durchläufe durch (II) und (III) gleichzeitig einen der Nachreaktion dienenden Zeitabschnitt.

Das im Beispiel der Fig. 1 den Wärmeaustauscher (III) verlassende noch weiter abgekühlte Reaktionsgemisch, dessen fühlbare Wärme insgesamt um den obengenannten Betrag von 2 bis 80°C abgenommen hat, wird nunmehr in die Teilströme (10) und (11) aufgeteilt. Der Teilstrom (10) umfaßt in der oben bereits beschriebenen Weise 80 bis 98 Gew.-% des gesamten Ablaufs vom Reaktor (I); der Teilstrom (11) stellt den Rest zu 100 % dar. Selbstverständlich ist es grundsätzlich möglich die Aufteilung von (9) in die Teilströme (10) und (11) an einer anderen Stelle des Verfahrensablaufes vorzunehmen.

Während der Teilstrom (10) zum Reaktoreingang zurückgeführt wird, wird der Teilstrom (11) der Aufarbeitung zugeführt. Hierzu gelangt (11) zunächst in einen Entspannungsbehälter (IV), worin eine Trennung in Flüssigkeit (12) und eine Gasphase (13) erfolgt. Die Gasphase (13) enthält überschüssiges CO₂, gegebenenfalls nicht vollständig umgesetztes POX und evtl. mit dem CO₂ und dem POX eingebrachte Inertgase. Je nach dem Anteil an Inertgasen wird die Gasphase (13) in die beiden Teilströme (14) und (15) aufgeteilt. (14) wird wieder in den Reaktor zurückgeführt, während (15) der Abgasentsorgung (25) zugeführt wird. Es ist selbstverständlich, daß bei höheren Inertgasgehalten ein höherer Teil von (13) in Form des Teilstromes (15) ausgeschleust wird und umgekehrt; einfache analytische Bestimmungen und Berechnungen sowie Vorversuche ergeben für den Fachmann ohne Schwierigkeiten das Optimum der Aufteilung der Gasphase (13).

Die in (IV) abgetrennte Flüssigkeit (12) wird nun in den Innenraum des Schnellverdampfers (II) geleitet. Der Schnellverdampfer (II) ist in bevorzugter Weise ein Dünnschichtverdampfer, ein Fallfilmverdampfer, ein Wendelrohrverdampfer oder ein Umlauf- oder Kletterfilmverdampfer. In (II) wird bei einem Vakuum von 2 bis 100 mbar, bevorzugt 4 bis 90 mbar, besonders bevorzugt 5 bis 80 mbar eine destillative Trennung in verdampftes PGC (16) und einen flüssigen Sumpfablauf (17) vorgenommen. Die Energie für diese destillative Trennung kann zum einen dem Reaktorablauf (8), der durch den Mantel oder die sonstige Heizeinrichtung des Schnellverdampfers (II) geleitet wird, entstammen, zum anderen der der flüssigen Phase (12) weiterhin innewohnenden fühlbaren Wärme.

Der Sumpfablauf (17) von (II) wird in die Teilströme (18) und (19) geteilt. Die weitaus größte Menge, beispielsweise 60 bis 98 %, bevorzugt 75 bis 97 %, besonders bevorzugt 90 bis 96 % der Gesamtmenge von (17) läuft als Teilstrom (18) wieder dem Reaktoreingang zu.

Sie enthält weiterhin den Katalysator. Damit schließt sich zudem der Kreislauf des im Reaktorsystem umgewälzten, als Reaktionsmedium dienenden PGC.

Der verbleibende Teilstrom (19) fließt einer Regenerierungsmöglichkeit (VI) für den Katalysator zu. Die Art der Regenerierung des Katalysators ist spezifisch auf den jeweils eingesetzten Katalysator zugeschnitten und ist dem Fachmann, sofern überhaupt eine Katalysatorregenerierung möglich ist, aus der obengenannten Literatur bekannt. Aus der Regenerierung (VI) kann ein Bruchteil von etwa unwirksam gewordenem Katalysator (20) ausgetragen werden. Der regenerierte Katalysator im Gemisch mit PGC (29) wird genau wie der Teilstrom (18) an den Reaktoreingang zurückgeführt. Die Regenerierung kann beispielsweise so durchgeführt werden, daß man Katalysatoren vom Typ der Gemische aus Alkalihalogeniden mit Halogeniden zweiwertiger Metalle, etwa NaBr/ZnBr₂, mit Halogenverbindungen, etwa HBr, behandelt.

Eine Ergänzung von verbrauchtem und ausgetragenem Katalysator durch frischen Katalysator (24) gelangt über (21) in den Reaktor.

Das den Innenraum von (II) verlassende abdestillierte PGC (16) wird in einem weiteren Wärmetauscher (V) unter Dampfgewinnung kondensiert und auf die gewünschte Temperatur abgekühlt. Ein solches kondensiertes PGC wird bei (23) abgezogen.

Etwaige gasförmige Anteile von (12), die mit verdampftem (16) nach (V) gebracht wurden, fallen in (V) bei der Kondensation von PGC als gasförmiger, über Kopf abzuziehender Strom an, der in die beiden Teilströme (22) und (26) geteilt wird. (22) wird der Abgasentsorgung (25) zugeführt, während (26) an den Reaktoreingang zurückgeführt wird. Die Aufteilung in die Teilströme (22) und (26) geschieht unter Anlegung ähnlicher Kriterien, wie dies weiter oben für die Teilströme (14) bzw. (15) bereits beschrieben wurde.

(27) ist das den Wärmeaustauschern (III) bzw. (V) zugeführte Wasser, das diesen Apparaten durch Ausnutzung der Verdampfungswärme oder fühlbaren Wärme als gewonnener Heizdampf (28) entnommen werden kann.

### Beispiel

In den bei 15 bar betriebenen Reaktor I gemäß Fig. 1 werden stündlich insgesamt über (1) 0,453 kg CO₂ und über (2) 0,569 kg POX eindosiert und zwar so, daß je 0,108 kg CO₂ beziehungsweise 0,142 kg POX als Gemisch durch die Leitungen (4) bis (7) fließen und 0,022 kg CO₂ dem über (10) mit einer Temperatur von ca. 141°C zurücklaufenden Reaktionsgemisch von etwa 30,2 kg vor Eintritt in den Reaktor I zugemischt (3) werden. Aus der Entspannung IV strömen weitere 0,03 kg Gas über (13) und (14) in den Reaktor I.

Nach Ablauf der Reaktion verlassen 31,8 kg Reaktionsgemisch mit einer Temperatur von ca. 159°C den Reaktor und fließen als Strom (8) in den Mantel des Schnellverdampfers II, den sie nach Beheizung der darin stattfindenden Destillation mit etwa 150°C verlassen, und als Strom (9) in den Wärmetauscher III, den sie nach Erzeugung von Wasserdampf mit etwa 140°C verlassen, und dann in den Strom (10) von ca. 30,2 kg und (11) von ca. 1,6 kg aufgeteilt werden. (10) kehrt, wie beschrieben, in den Reaktor zurück, während (11) in IV auf 1 bar entspannt wird, wobei die gasförmige Phase (13) von 0,03 kg nach I und die flüssige Phase (12) von 1,57 kg in den Schnellverdampfer II geleitet und dort bei etwa 50 mbar in ca. 1,03 kg dampfförmiges PGC (16) und ca. 0,54 kg Sumpf (17) gespalten wird. Das dabei entweichende Restgas von etwa 0,03 kg wird über (22) und (25) in die Abgasentsorgung abgezogen. PGC (16) kondensiert im Wärmetauscher V unter Wasserdampferzeugung.

Nach Aufteilung des Sumpfes (17) entsprechend ca. 9:1 fließt die größere Menge von ca. 0,49 kg als Strom (18) und (21) nach I und die kleinere von ca. 0,05 kg (19) in die Regenerierung VI, die sie nach Behandlung mit einer sehr geringen Menge einer Halogenverbindung als (29) wieder verläßt und als (21) zurück in den Reaktor geleitet wird.

## Patentansprüche

1. Verfahren zur katalytischen Herstellung von Propylenglykolcarbonat (PGC) durch Umsetzung von Propylenoxid (POX) und Kohlendioxid in PGC als Reaktionsmedium bei erhöhter Temperatur und erhöhtem Druck und Abtrennung des gebildeten PGC vom Katalysator, dadurch gekennzeichnet, daß
a) das Verfahren kontinuierlich und adiabat bei einem Druck von 2 bis 200 bar, bevorzugt 5 bis 80 bar, besonders bevorzugt 8 bis 60 bar und innerhalb eines Temperaturbereiches von 110 bis 200°C, bevorzugt 110 bis 190°C, besonders bevorzugt 110 bis 180°C bei einer adiabaten Temperatursteigerung von 2 bis 80°C durchgeführt wird, wobei die Eingangstemperatur so gewählt wird, daß die adiabate Temperatursteigerung innerhalb des genannten Temperaturbereiches bleibt,
b) das als Reaktionsmedium dem Reaktor pro Zeiteinheit zulaufende PGC das 7- bis 350-fache des in dieser Zeiteinheit gebildeten PGC beträgt.
c) 1,01 bis 1,5 Mol, bevorzugt 1,01 bis 1,4 Mol, besonders bevorzugt 1,01 bis 1,35 Mol Kohlendioxid pro Mol POX eingesetzt werden und an allen Stellen des Reaktors ein Kohlendioxid-Überschuß aufrechterhalten wird und
d) 80 bis 98 Gew.-%, bevorzugt 85 bis 97 Gew.-%, des gesamten Reaktionsgemisches an den Eingang des Reaktors zurückgeführt werden und der Rest auf PGC aufgearbeitet wird.

2. Verfahren nach Anspruch 1, dadurch gekennzeichnet, daß im Reaktor eine intensive Durchmischung aufrechterhalten wird.

3. Verfahren nach Anspruch 2, dadurch gekennzeichnet, daß der Reaktor Lochböden, Rohrverteiler, Zweistoffdüsen, Strahldüsen, Düsen, geschlossene Gasverteiler, Impingment Aerator-Elemente, Mischerelemente oder eine Kombination mehrerer von ihnen enthält.

4. Verfahren nach Anspruch 1, dadurch gekennzeichnet, daß zur Erzeugung des Kohlendioxid-Überschusses mindestens ein Teil des Kohlendioxids vor der Eindüsung des POX in das als Reaktionsmedium dienende PGC eingespeist wird, wobei bevorzugt dieses Kohlendioxid dem PGC vor Eintritt in den Reaktor zugesetzt wird.

5. Verfahren nach Anspruch 4, dadurch gekennzeichnet, daß POX allein oder als Gemisch mit gegebenenfalls restlichem Kohlendioxid an mindestens zwei aufeinanderfolgenden Stellen so in den Reaktor eingespeist wird, daß stets ein Kohlendioxid-Überschuß besteht.

6. Verfahren nach Anspruch 5, dadurch gekennzeichnet, daß der vorgesehene Überschuß an Kohlendioxid dem PGC vor dessen Eintritt in den Reaktor zugesetzt wird und das restliche Kohlendioxid als äquimolares POX/CO₂-Gemisch in den Reaktor eingespeist wird.

7. Verfahren nach Anspruch 1, dadurch gekennzeichnet, daß die erreichte fühlbare Wärme des Reaktionsgemisches für die Aufarbeitung des Reaktionsgemisches zu PGC und/oder bei der Erzeugung von Heizdampf und/oder bei der Durchführung endothermer Verfahren genutzt werden kann.

8. Verfahren nach Anspruch 7, dadurch gekennzeichnet, daß bei der Gewinnung der fühlbaren Wärme eine Abkühlung des Reaktionsgemisches um 2 bis 80°C eintritt.

9. Verfahren nach Anspruch 7, dadurch gekennzeichnet, daß die Aufarbeitung zu PGC unter Nutzung der gewonnenen fühlbaren Wärme durchgeführt wird.

10. Verfahren nach Anspruch 9, dadurch gekennzeichnet, daß die Aufarbeitung destillativ bei 2 bis 100 mbar, bevorzugt bei 4 bis 90 mbar, besonders bevorzugt bei 5 bis 80 mbar durchgeführt wird.

## Claims

1. Process for the catalytic preparation of propylene glycol carbonate (PGC) by reacting propylene oxide (POX) and carbon dioxide in PGC as reaction medium at elevated temperature and elevated pressure and separating off from the catalyst the PGC formed, characterized in that
a) the process is carried out continuously and adiabatically at a pressure of 2 to 200 bar, preferably 5 to 80 bar, particularly preferably 8 to 60 bar and within a temperature range of 110 to 200°C, preferably 110 to 190°C, particularly preferably 110 to 180°C with an adiabatic temperature increase of 2 to 80°C, the entry temperature being selected so that the adiabatic temperature increase remains within the temperature range mentioned,
b) the PGC flowing into the reactor as reaction medium per unit of time is 7 to 350 times the PGC formed in this unit of time,
c) 1.01 to 1.5 mol, preferably 1.01 to 1.4 mol, particularly preferably 1.01 to 1.35 mol of carbon dioxide are used per mole of POX and at all points of the reactor a carbon dioxide excess is maintained and
d) 80 to 98 % by weight, preferably 85 to 97 % by weight, of the total reaction mixture are returned to the entrance of the reactor and the remainder is worked up to give PGC.

2. Process according to Claim 1, characterized in that intensive mixing is maintained in the reactor.

3. Process according to Claim 2, characterized in that the reactor contains perforated trays, pipe distributors, two-component nozzles, jet nozzles, nozzles, closed gas distributors, impingement aerator elements, mixer elements or a combination of a plurality thereof.

4. Process according to Claim 1, characterized in that, to generate the carbon dioxide excess, at least some of the carbon dioxide is fed into the PGC serving as reaction medium before the injection of the POX, this carbon dioxide being preferably added to the PGC before entry into the reactor.

5. Process according to Claim 4, characterized in that POX, alone or as a mixture with any remaining carbon dioxide, is fed into the reactor at at least two sequentially following points in such a way that a carbon dioxide excess always exists.

6. Process according to Claim 5, characterized in that the excess of carbon dioxide provided is added to the PGC before its entry into the reactor and the remaining carbon dioxide is fed into the reactor as an equimolar POX/CO₂ mixture.

7. Process according to Claim 1, characterized in that the sensible heat achieved of the reaction mixture can be used for the work-up of the reaction mixture to give PGC and/or can be used in the generation of heating steam and/or can be used in carrying out endothermic processes.

8. Process according to Claim 7, characterized in that, in the recovery of the sensible heat, a cooling of the reaction mixture by 2 to 80 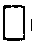 C takes place.

9. Process according to Claim 7, characterized in that the work-up to give PGC is carried out with utilization of the recovered sensible heat.

10. Process according to Claim 9, characterized in that the work-up is carried out by distillation at 2 to 100 mbar, preferably at 4 to 90 mbar, particularly preferably at 5 to 80 mbar.

## Revendications

1. Procédé de préparation catalytique de carbonate de propylène glycol (PGC) par conversion d'oxyde de propylène (POX) et de dioxyde de carbone en PGC servant de milieu de réaction, à température élevée et pression élevée, et avec séparation du PGC produit et du catalyseur, lequel procédé est caractérisé en ce que :
a) le procédé est conduit en continu et dans les conditions adiabatiques, à une pression de 2 à 200 bars, de préférence de 5 à 80 bars, de manière particulièrement préférable de 8 à 60 bars, et à l'intérieur d'une plage de température de 110 à 200 °C, de préférence de 110 à 190 °C et de manière particulièrement préférable de 110 à 180 °C, avec une augmentation adiabatique de la température de 2 à 80 °C, la température de départ étant sélectionnée de telle sorte que l'augmentation adiabatique de la température reste à l'intérieur de ladite plage de température,
b) le PGC entrant par unité de temps dans le réacteur pour y servir de milieu de réaction représente de 7 à 350 fois le PGC formé pendant cette unité de temps,
c) de 1,01 à 1,5 mole, de préférence de 1,01 à 1,4 mole et de manière particulièrement préférable de 1,01 à 1,35 mole de dioxyde de carbone sont utilisées par mole de POX, et à tout endroit du réacteur, on maintient un excès en dioxyde de carbone et
d) de 80 à 90 % en poids, de préférence de 85 à 97 % en poids de l'ensemble du mélange de réaction sont renvoyés à l'entrée du réacteur, et le reste est traité pour en récupérer le PGC.

2. Procédé selon la revendication 1, caractérisé en ce que l'on maintient dans le réacteur un mélange intime intense.

3. Procédé selon la revendication 2, caractérisé en ce que le réacteur contient des fonds perforés, des répartiteurs tubulaires, des gicleurs à deux matières, des gicleurs de projection, des gicleurs, des répartiteurs de gaz fermés, des éléments d'aérateur à percussion, des éléments mélangeurs ou une combinaison de plusieurs de ceux-ci.

4. Procédé selon la revendication 1, caractérisé en ce que pour créer un excès de dioxyde de carbone, au moins une partie du dioxyde de carbone est introduite dans le PGC servant de milieu de réaction avant l'injection du POX, et ce dioxyde de carbone est ajouté au PGC de préférence avant son entrée dans le réacteur.

5. Procédé selon la revendication 4, caractérisé en ce que le POX est introduit dans le réacteur seul ou en tant que mélange avec du dioxyde de carbone résiduel éventuel, en au moins deux points successifs, de telle sorte qu'il existe toujours un excès de dioxyde de carbone.

6. Procédé selon la revendication 5, caractérisé en ce que l'excès prévu en dioxyde de carbone est ajouté au PGC avant son entrée dans le réacteur, et le reste du dioxyde de carbone est introduit dans le réacteur sous la forme d'un mélange équimolaire de POX/CO₂.

7. Procédé selon la revendication 1, caractérisé en ce que la chaleur sensible que le mélange de réaction atteint peut être valorisée pour la poursuite du traitement du mélange de réaction en PGC et/ou pour la création de vapeur chaude et/ou pour la mise en oeuvre de procédés endothermiques.

8. Procédé selon la revendication 7, caractérisé en ce que lors de la récupération de la chaleur sensible, il se produit un refroidissement de 2 à 80 °C du mélange de réaction.

9. Procédé selon la revendication 7, caractérisé en ce que la transformation en PGC est réalisée avec réutilisation de la chaleur sensible récupérée.

10. Procédé selon la revendication 9, caractérisé en ce que le retraitement s'effectue par distillation entre 2 et 100 mbars, de préférence entre 4 et 90 mbars et de manière particulièrement préférable entre 5 et 80 mbars.
